# EUROPEAN PATENT APPLICATION

(11) **EP 0 638 309 A1**
(43) Date of publication of application: **15.02.1995**
(21) Application number: 94110805.2
(22) Date of filing: 12.07.1994
(51) Int. Cl.: A61K 31/00, A61K 31/12, A61K 31/35, A61K 31/71, A61K 31/505, A61K 31/20, A61K 31/47

(54) **Substances inhibiting proteic adp ribosilation suitable to prevent the diabetes mellitus complications**

(30) Priority: 14.07.1993 IT MI931554
(71) Applicant: ISTITUTO BIOCHIMICO ITALIANO GIOVANNI LORENZINI S.p.A., I-20141 Milano (IT)
(72) Inventor: Gorio, Alfredo, I-20124 Milan (IT); Borella, Fabio, I-20133 Milan (IT)
(74) Representative: Gervasi, Gemma, Dr.

(57) **Abstract**

The present invention refers to the use of substances inhibiting proteic ADP-ribosilation and of their soluble salts and complexes as active substances for the preparation of pharmaceutical compositions suitable to prevent the diabetes mellitus complications as neuropathies, nephropathies, retinopathies, macroangiopathies, microangiopathies and hepatopathies.

## Description

### PRIOR ART

The diabetes mellitus is a well known syndrome characterized by hyperglycemia, glycosuria, polyuria and polydipsia and by a series of serious complications as neuropathies, nephropathies, retinopathies, macroangiopathies, microangiopathies and metabolic alterations to the hepatic level.

The diabetes mellitus therapy is based on the diet and on the treatment by insulin and by oral hypoglycemizing drugs (L. S. Goodman and A. Gilman, The Pharmac. Basis of Therapeutics, third edition, page 1600).

However, the therapeutical results, even allowing to restrain the glycemia, are often unsatisfactory as far as the complications prevention.

On the other side it is known that in some pathologies the proteic ADP-ribosilation process is stimulated. In particular this process is stimulated by the cholera and the whooping-cough toxins as reported for example by R.S. Duman in J. of Neuroch. 57.6, pagg. 2124-2132, 1991.

Moreover, some further endogenous substances able to promote the proteic ADP-ribosilation as for example the NADPH, the arginine, the nitrogen monoxide and the corticosteroids are known (Stamler et al., Nature, pag. 1898-1902, vol. 258, Dec. 18,1992).

In addition a lot of substances able to inhibit the proteic ADP-ribosilation are known, as it is shown for example by M. Banasik et al. in J. Biol Chem. 267,3, January 25, 1992, pag. 1569-1575.

### SUMMARY

We have found and constitutes the main object of the present invention, that the substances inhibiting the proteic ADP-ribosilation, and their soluble salts and complexes may be successfully used as active substances for preparing pharmaceutical compositions suitable to prevent the diabetes mellitus complications.

In particular said pharmaceutical compositions are effective in preventing the neuropathies, the nephropathies, the retinopathies, the macroangiopathies, the microangiopathies and the hepatopathies following the diabetes mellitus.

Therefore the present invention refers also to the therapeutical method for preventing said complications consisting in administering the present invention compositions to the patients suffering from diabetes mellitus.

### DETAILED DESCRIPTION OF THE INVENTION

The characteristics and the advantages of the use of the substances inhibiting proteic ADP-ribosilation, their soluble salts and their complexes for preparing pharmaceutical compositions suitable to prevent the diabetes mellitus complications, will he more evident during the following detailed description.

Suitable substances to be used according to the present invention are, for example, vitamin K1, vitamin K2, vitamin K3, 1.8-naphthalimide, 2-nitro-6(5H)-phenanthridinone, 4-amino-1.8-naphthalimide, benzoyleneurea, flavone, novobiocin, folic acid, arachidic acid, stearic acid, palmitic acid, myristic acid, linoleic acid (C 18:2-cis-9.12), linolenic acid (C18:3-cis-9.12.15) and silybin, as well as their soluble salts and their complexes.

The description includes method and results of an experimentation in vitro, method and results of an experimentation in vivo and the information for the pharmaceutical compositions preparation and for the therapeutical method.

### Experimentation in vitro

A diabetic neuropathy in vitro model has been carried out using neuroblastoma cells cultured in chronic hyperglucosemia conditions. This condition causes the typical diabetes mellitus dismetabolic effects as the polyols (sorbitol) accumulation, the myoinositol reduction and the reduction of the Na pump functionality. Cells of 5 YSY neuroblastoma have been cultured in standard conditions as described in "A Dissection and Tissue Culture Manual of the Nervous System" - Ed. A. Shahar et al. - Alan R., Liss. Inc., 1989.

Four experimental groups have been carried out:
1) cells grown in normal medium (control);
2) cells grown in glucose;
3) cells grown in glucose plus a substance inhibiting proteic ADP-ribosilation;
4) cells grown in glucose plus a substance non inhibiting proteic ADP-ribosilation and used in clinic treatment of the diabetes mellitus complications (comparison).

In the groups from 2) to 4) glucose (30 mM) has been added to the culture renewing the culture medium and then adding glucose every 72 hours, during a three weeks period.

In the group 3), at the same time of the glucose, one of the substances inhibiting proteic ADP-ribosilation reported in the table 1 (amount 10⁻⁵ M) has been added.

In the group 4), at the same time of the glucose one of the substances non inhibiting proteic ADP-ribosilation reported in the table 1 (dermatan sulphate and tolrestat) in amount 10⁻⁵ M has been added.

Subsequently retinoic acid (5 x 10⁻⁶ M) has been added to the cultures 1) to 4) for causing the cells neuritogenesis which has been valued by the observation at the optic microscope considering as neurit the cytoplasmatic extension having a length corresponding at least to twice as the diameter of the cell.

The results of the experimentation are shown in the table 1.

The cells of the experimental group 1) (control) show at 100% at least one neurit after 12 days from the retinoic acid addition.

In the cells of the experimental group 2), for the dismetabolic action caused by the glucose, a potent inhibition of the neuritogenesis process which shows a situation of diabetic neuropathy has been observed.

In the experimental group 3) (addition of glucose and substances inhibiting proteic ADP-ribosilation) it is observed that the added substance allows to prevent significantly, and sometimes completely, the diabetic neuropathy.

In the experimental group 4) (addition of glucose and substances non inhibiting proteic ADP-ribosilation) it is observed that the added substance does not exercise any effect for preventing the diabetic neuropathy, although both the dermatansulphate and the tolrestat have a clinic use in the diabetes mellitus complications treatment.

**TABLE 1**

| Percentage of cells with neurits after retinoic acid addition. | | | | |
|---|---|---|---|---|
| Days in retinoic acid | | 3 | 7 | 12 |
| Group 1: | Control | 46 | 67 | 100 |
| Group 2: | Glucose | 28 | 36 | 50 |
| Group 3: | Glucose + Vit K1 | 48 | 66 | 97 |
| | Glucose + Vit K2 | 45 | 65 | 98 |
| | Glucose + Vit K3 | 39 | 54 | 80 |
| | Glucose + 1.8 Naphthalimide | 46 | 66 | 100 |
| | Glucose + 2-nitro-(6(5H)-phenanthridinone | 40 | 58 | 89 |
| | Glucose + 4-amino-1.8-naphthalimide | 35 | 50 | 81 |
| | Glucose + benzoyleneurea | 36 | 48 | 66 |
| | Glucose + flavone | 38 | 56 | 74 |
| | Glucose + novobiocin | 38 | 42 | 68 |
| | Glucose + arachidic acid | 44 | 69 | 100 |
| | Glucose + stearic acid | 48 | 66 | 100 |
| | Glucose + palmitic acid | 53 | 71 | 100 |
| | Glucose + myristic acid | 38 | 58 | 81 |
| | Glucose + linoleic acid (C18:2-cis-9.12) | 34 | 52 | 86 |
| | Glucose + linolenic acid (C18:3-cis-9.12.15) | 39 | 48 | 79 |
| Group 4: | Glucose + dermatansulphate | 24 | 35 | 48 |
| | Glucose + tolrestat | 26 | 34 | 45 |

On the experimental groups above described also the myoinositol metabolism alteration has been studied which has been valued by the method described by Yorek et al., in "Diabetes", pag. 240-248, vol. 40 (1991).

The results are shown in the table 2 where the myoinositol amount accumulated after the incubation with the marked substance is meant with "accumulation", and the myoinositol fraction linked to the membranes is meant with "incorporation".

**TABLE 2**

| Myoinositol metabolism | | | |
|---|---|---|---|
| Experimental group | | Accumulation n mol/µg of protein | Incorporation n mol/µg of protein |
| Gr. 1: | Control | 9.62 | 0.94 |
| Gr. 2: | Glucose | 1.89 | 0.31 |
| Gr. 3: | Glucose + Vit K1 | 9.66 | 0.98 |
| | Glucose + Vit K2 | 8.99 | 0.88 |
| | Glucose + Vit K3 | 8.56 | 0.85 |
| | Glucose+1.8-naphthalimide | 9.20 | 0.91 |
| | Glucose + 2-nitro-(6(5H)-phenanthridinone | 8.51 | 0.81 |
| | Glucose + 4-amino-1.8-naphthalimide | 8.01 | 0.78 |
| | Glucose + benzoyleneurea | 6.91 | 0.69 |
| | Glucose + flavone | 6.54 | 0.67 |
| | Glucose + novobiocin | 6.0 | 0.60 |
| | Glucose + arachidic acid | 9.02 | 0.92 |
| | Glucose + stearic acid | 9.89 | 0.95 |
| | Glucose + palmitic acid | 9.39 | 0.89 |
| | Glucose + myristic acid | 8.91 | 0.85 |
| | Glucose + linoleic acid (C18:2-cis-9.12) | 8.66 | 0.88 |
| | Glucose + linolenic acid (C18:3-cis-9.12.15) | 8.44 | 0.81 |
| Gr. 4: | Glucose + dermatansulphate | 1.79 | 0.29 |

Comparing the table 1 with the table 2 we can observe that the data relative to the neuritogenesis and to the myoinositol metabolism result in perfect agreement and show that the substances able to inhibit the proteic ADP-ribosilation are active in preventing the experimental diabetes mellitus effects. The same data suggest also a power correlation because the most effective agents inhibiting the proteic ADP-ribosilation are the most effective also in preventing the experimental diabetes mellitus effects.

### Experimentation in vivo

The effectiveness verification of the substances inhibiting proteic ADP-ribosilation in preventing the diabetes mellitus complications has been carried out in vivo on rats in diabetes mellitus models induced by alloxan. With this purpose, a first experiment has been performed on the substance P axonal transport in the five following experimental groups, each consisting of N° 8 rats:
1) control group;
2) diabetic rats group;
3) diabetic rats group treated with vitamin K1 (substance inhibiting the proteic ADP-ribosilation);
4) diabetic rats group treated with arachidic acid (substance inhibiting the proteic ADP-ribosilation);
5) diabetic rats group treated with dermatan sulphate (substance non inhibiting proteic ADP-ribosilation).

The Sprague Dawley strain rats used, 250 gr weight, male sex, were supplied by Charles River.

The diabetes has been induced in the rats of the groups from 2) to 5) by a single subcutaneous injection of 100 mg/kg of alloxan dissolved in pH 4.5 0.1 M citrate-phosphate buffer solution.

The control group has been treated with a single subcutaneous injection of physiologic solution.

The pharmacological treatment of the groups 3) and 5) respectivelly with vitamin K1 and dermatansulphate, has been carried out by intraperitoneal administration of said substances dissolved in physiologic solution at 10 mg/kg dose. The treatment of the group 4) has been carried out by oral administration of 10 mg/kg of arachidic acid in suspension in physiologic solution. Groups 1) and 2) have been injected with an equivalent volume of physiologic solution.

The pharmacological treatment started 7 days after the diabetes induction and prosecuted until the sacrifice with daily administration.

To estimate the axonal transport of substance P the accumulation of the substance P in three segments of sciatic nerve two millimeters long was estimated 24 hours from the ligature of the same nerve.

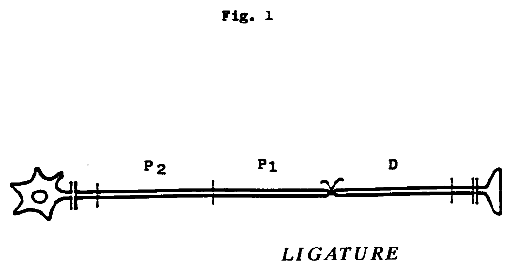

Two of said segments (P₁ and P₂) were proximal segments and one of said segments (D) was distal segment, as shown in fig.1.

The operations of the sciatic nerve ligature, of the tissues withdrawal from the same and of substance P extraction from the tissues for the radioimmunologic dosage, have been carried out by the following way.

The animals were anaesthetised by intraperitoneal administration of 35 mg/kg of pentobarbital dissolved in 0.9% physiologic solution. After wright thigh epilation and cutis section the sciatic nerve was exposed at half thigh level (at least 0.8 cm from the popliteal cavity where the nerve ramifies). The ligature has been made by sterile silk thread which has been twice knotted to about half of the exposed nerve portion.

Finally the section has been closed by a suture with internal points of pure silk thread, applying some antibiotic on the wound by painting and finally suturing the cutis by 3 or 4 external metallic points.

The animals sacrifice has been made, in every experimental model, by guillotining.

The sacrifice has been accomplished after 8 weeks from the diabetes induction, after 24 hours from the ligature.

Immediately after the sacrifice, in the former operation seat, the removal of the whole sciatic nerve has been made and finally from this the three segments each about 2 mm long have been withdrawn:
segment P1 (proximal contiguous to the ligature);
segment P2 (immediately above P1) and
segment D (distal contiguous to the ligature),
as it is shown in fig. 1.

Subsequently the whole contralateral sciatic nerve has been withdrawn.

The withdrawn tissues have been placed in polypropylene test-tubes held in ice and containing a volume of acetic acid 1 N in 10:1 ratio with the tissue weight. Then the tissues homogenization has been done by a continuous speed variation homogenizer (Heavy Stirrer, mod. 12) and a polytetrafluoroethylene pestle perfectly adherent to the test-tube walls. After the homogenate aliquots required for the tissue proteins dosage have been withdrawn (0.1 ml) the test-tubes have been immerged in a boiling water bath for 8-10 minutes, for inactivating the peptidases eventually still present in the samples, and then left for about 30 minutes at 4 °C for completely cooling.

The buffy coat obtained by centrifugation of the homogenate at 40.000 x g for 10 minutes, has been lyophilized and the dry residue has been reconstituted in distilled water, stirred by vortex and centrifuged at 40.000 x g for 10 minutes. From the samples so reconstituted the suitable aliquots required for the substance P radioimmunological dosage have been withdrawn.

The samples relative to the segments P1 and P2 have been used for estimating the anterograde axonal transport while the samples relative to the segment D have been used for estimating the retrograde axonal transport.

In each animal the substance P content in the contralateral non linked sciatic nerve (NSc) has been evaluated too.

The substance P accumulation is an estimate of the axonal transport and the base value of reference derives from the NSc contents measurement.

The table 3 shows, for each experimental group, the substance P contents in the various withdrawn segments and in the contralateral nerve, expressed in ng/mg of tissular protein ± ES. Analizing the data it is noticed that in all the three segments of the sciatic nerve of the control group animals there was substance P accumulation, of about 6 times in the segment P1 and of about 2.5 times in the segment P2.

The retrograde transport is represented by the substance P increase in the segment D. In the diabetic animals group there is not accumulation in the segment D (deficit of retrograde transport) and a reduced accumulation in the segment P1 (deficit of anterograde transport). In the groups of the diabetic animals respectivelly treated with vitamin K1 and arachidic acid the anterograde and retrograde accumulation is normalized, indicating that these substances are active in preventing the diabetes neurological complications.

**TABLE 3**

| - The substance P content in the various segments of the sciatic nerve submitted to ligature and in the contralateral sciatic nerve (a). | | | | | |
|---|---|---|---|---|---|
| Segment | Group 1) (control) | Group 2) (diabetics) | Group 3) (diabetics treated with vitamin K1) | Group 4) (diabetics treated with arachidic acid) | Group 5) (diabetics treated with dermatansulphate) |
| NSc | 1.24±0.10 | 1.28±0.007 | 1.28±0.008 | 1.24±0.006 | 1.20±0.009 |
| P1 | 8.24±0.280 | 4.64±0.086 | 8.72±0.16 | 9.44±0.14 | 3.76±0.0093 |
| P2 | 3.00±0.06 | 1.12±0.04 | 2.44±0.09 | 1.40±0.05 | 1.24±0.008 |
| D | 1.84±0.08 | 1.16±0.04 | 1.96±0.08 | 1.80±0.06 | 1.12±0.009 |

(a) The values are expressed in ng/mg of tissular protein ± ES.

A second experiment on the substance P axonal transport has been made in the three following experimental groups, each consisting of 6 rats:
1a) control group;
2a) diabetic rats group;
3a) diabetic rats group treated with silybin (substance inhibiting proteic ADP-ribosilation belonging to the flavon class).

The experiment has been carried out according to the modalities formerly described using for the pharmacological treatment the silybin which has been administered to the group 3a) rats by oral route at 50 mg/kg/die dose, in aqueous suspension.

The obtained results are shown in the table 4).

**TABLE 4**

| - The substance P content in the various segments of the sciatic nerve submitted to ligature and in the contralateral sciatic nerve (a). | | | |
|---|---|---|---|
| Segment | Group 1a) (controls) | Group 2a) (diabetics) | Group 3a) (diabetics treated with silybin) |
| NSc | 2.29±0.13 | 2.23±0.096 | 2.26±0.040 |
| P1 | 12.14±0.35 | 6.67±0.24 | 11.42±0.18 |
| P2 | 4.54±0.12 | 3.38±0.11 | 7.07±0.59 |
| D | 2.53±0.14 | 2.79±0.067 | 4.05±0.071 |

(a) The values are expressed in ng/mg of tissular protein ± ES.

The table 4 results constitute a confirmation of the fact that the substances inhibiting proteic ADP-ribosilation are effective in preventing the diabetes neurological complications.

A further experimentation on the silybin effect on the ADP-ribosilation of retina proteins G has been carried out. The evaluation of said proteins ribosilability has been performed by the method described by Duman et al. in J. Neurochem. (1991). pages 2124-2132.

For said experimentation the retinas withdrawn from the rats of the groups 1a), 2a) and 3a) used for the experimentation on the substance P axonal transport, were used.

The retinas have been homogenized and subdivided in cellular fractions and the membrane fraction has been selected for the ADP-ribosilation reaction in vitro.

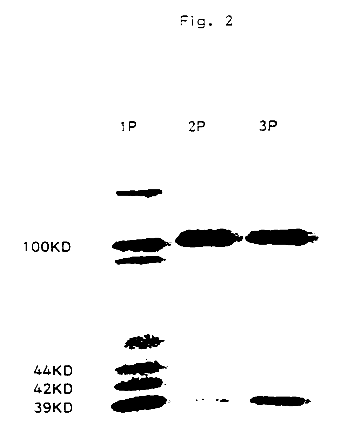

The ADP-ribosilation results are reported in fig. 2 which represents the autoradiogram of an electrophoretic gel obtained from said membrane cellular fraction.

Column 1P derives from the retinas of the control group rats, column 2P derives from the retinas of the diabetic rats and column 3P derives from the retinas of the diabetic rats treated with silybin at a 50 mg/kg dose.

Column 1P of the autoradiogram shows that five proteic bands are ribosilated, two of which having a molecular weight higher than 100 KD and the remaining having respectively a molecular weight of 44 KD, 42 KD and 39 KD. The 39 KD band is due to the transducin as demonstrated by immunological analysis.

As known, the transducin is the essential protein for the photoreceptor functionality in that it allows the transduction of the photonic luminous signal.

In the diabetic rats (column 2P) the ribosilation in vitro of the highest molecular weight band is evident whereas for the other bands the ribosilation results in a decrease. In particular the transducin band results in a decrease. Said result shows that in the diabetic rats retina an endogenous proteic ribosilation process occurs by which the ribosilability results in a decrease.

On the contrary, in the diabetic rats treated with silybin (column 3) one remarks that the transducin ribosilability is maintained.

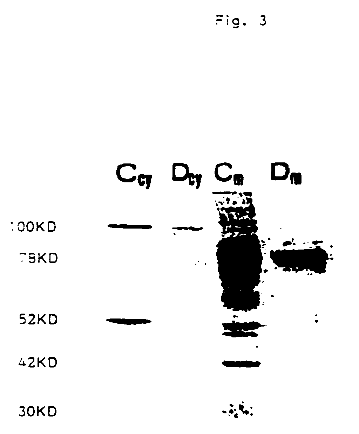

Therefore it is possible to conclude that in the diabetic retinopathy a process of transducin ADP-ribosilation occurs, this process being avoided by administration of a substance inhibiting proteic ADP-ribosilation as for example silybin. Finally we have performed a study on human sural nerve biopsies and discovered that the alteration of the proteic ADP-ribosilation is a process which occurs also in the diabetic patients nerves. As shown in the autoradiogram of the figure 3 some proteins are selectively ADP-ribosilated both in the cytoplasmatic fraction (cy) and in the membrane fraction (m) of the nerve.

The ADP-ribose incorporation is markedly reduced in the sural nerve biopsy from a diabetic patient (D) with respect to that observed in the sural nerve biopsy from an healthy patient (C) amputated of the lower limb for an accident.

The data analysis shows for example a very marked difference in the nerve membrane fraction. In the column Cm (healthy patient, membrane fraction) the incorporation of ADP-ribose is evident in proteic bands having molecular weight of 100, 60, 52, 51, 42 and 30 KD. In particular said incorporation is very high in a band from 92 to 76 KD. In the sural nerve membranes of the diabetic patient (Dm) the ADP-ribose incorporation is markedly reduced in all the above cited bands.

These data show that proteic ADP-ribosilation is a functional marker of experimental as well as of human diabetes.

In addition it is to remark that the substances inhibiting proteic ADP-ribosilation have not the capability to control the glycemia and the body weight therefore carrying on an activity type different from the insulin one.

The results of weight and glycemia control in the 5 experimental groups of the formerly described first experiment in vivo are shown in the table 5. The blood samples have been withdrawn at the caudal vein level before the animals sacrifice.

**TABLE 5**

| Body weight and glycemia in diabetic animals 8 weeks after the diabetes induction. | | | |
|---|---|---|---|
| Experimental groups | | Body weight (gr.) | Glycemia (mg/dl) |
| Group 1: | controls | 448±7.23 | 117±2 |
| Group 2: | diabetics | 226±0.84 | 458±4.2 |
| Group 3: | diabetics treated with vitamin K1 | 244±12.6 | 438±6.4 |
| Group 4: | diabetics treated with arachidic acid | 239±12.6 | 471±9.4 |
| Group 5: | diabetics treated with dermatansulphate | 229±12.9 | 468±8.2 |

The substances of the present invention in addition to carrying on the explained activity, have the characteristic to be practically without toxic effects.

Thanks to their characteristics, the substances inhibiting proteic ADP-ribosilation according to the present invention find an important therapeutical application in the diabetes mellitus treatment for preventing the relative complications including the neuropathies, the nephropathies, the retinopathies, the microangiopathies, the macroangiopathies and the hepatopathies.

Therefore the present invention refers to use of the substances inhibiting proteic ADP-ribosilation and their soluble salts and their complexes for preparing pharmaceutical compositions comprising effective amounts of said substances in mixture with pharmacologically acceptable diluents and excipients, suitable to the preventive treatment of the diabetes mellitus complications by oral or parenteral administration.

As complexes of the present invention substances are intended, for example, their complexes with cyclodextrins, polyvinylpyrrolidone, liposomes, phospholipids in general, polysaccharides and other complexing substances suitable to increase the bioavailability of the active substances.

Compositions examples according to the present invention are as follows:

### EXAMPLE 1

Hard gelatine perle containing 10 mg of active substance

| | |
|---|---|
| Active substance | 10 mg |
| Lactose | 62 mg |
| Maize starch | 27 mg |
| Magnesium stearate | 1 mg |

### EXAMPLE 2

Coated tablet containing 250 mg of active substance

| | |
|---|---|
| Active substance | 250 mg |
| Lactose | 62 mg |
| Maize starch | 24 mg |
| Magnesium stearate | 8 mg |
| Aerosil | 10 mg |
| Dextrose | 20 mg |
| Avicel | 12 mg |
| Crosslinked polyvinylpyrrolidone | 14 mg |
| Talc | 10 mg |

Coating by methacrylic polymers:

| | |
|---|---|
| Eudragit 30 D | 8 mg |
| Titanium dioxide | 0.4 mg |
| Dibutylphthalate | 0.2 mg |
| Talc | 0.6 mg |

### ESAMPLE 3

Drinking small bottle containing 500 mg of active substance

| | |
|---|---|
| Active substance | 500 mg |
| Carboxymethylcellulose | 30 mg |
| Nipagin | 10 mg |
| Aspartame | 20 mg |
| Mint flavour | 3 mg |
| Anise flavour | 5 mg |
| Water | 10 mg |

### EXAMPLE 4

Vials containing from 1 to 200 mg of active substance:
a) ready in distilled water. 2 - 3 ml
b) lyophilized + solvent.

Moreover the invention refers also to the therapeutical method to be applied in human therapy in the preventive treatment of the diabetes mellitus complications, comprising the administration by oral or parenteral route of a pharmacologically effective dose of a substance inhibiting proteic ADP-ribosilation.

The amount of substance inhibiting proteic ADP-ribosilation to be administered for the therapeutical treatment is different from substance to substance being comprised between 10 and 500 mg/dose for the oral administration and from 1 to 200 mg/dose for the parenteral administration.

## Claims

1. Use of substances inhibiting proteic ADP-ribosilation, their soluble salts and their complexes, as active substances for the preparation of pharmaceutic compositions suitable to prevent the diabetes mellitus complications.

2. Use as claimed in claim 1, characterized in that said substances inhibiting proteic ADP-ribosilation are selected from the group comprising Vitamin K1, Vitamin K2, Vitamin K3, 1.8-Naphthalimide, 2-nitro-6(5H)-phenanthridinone, 4-amino-1.8-naphthalimide, Benzoyleneurea, Flavone, Novobiocin, Folic acid, Arachidic acid, Stearic acid, Palmitic acid, Myristic acid, Linoleic acid (C18:2-cis-9.12), Linolenic acid (C18:3-cis-9.12.15) and Silybin.

3. Use as claimed in claim 1, characterized in that said diabetes mellitus complications comprise neuropathies, nephropathies, retinopathies, microangiopathies, macroangiopathies and hepatopathies.

4. Pharmaceutical compositions for preventing diabetes mellitus complications containing as active substance an effective dose of a substance inhibiting proteic ADP-ribosilation in a mixture with pharmacologically acceptable diluent and excipient substances.

5. Compositions as claimed in claim 4, in a form suitable to oral administration.

6. Compositions as claimed in claim 4, in a form suitable to parenteral administration.

7. Therapeutic method for the preventive treatment of diabetes mellitus complications comprising the administration of an effective dose of a substance inhibiting proteic ADP-ribosilation.

8. Method as claimed in claim 7, characterized in that said administration is carried out by oral route with an amount of substance inhibiting proteic ADP-ribosilation between 10 and 500 mg/dose.

9. Method as claimed in claim 7, characterized in that said administration is carried out by parenteral route with an amount of substance inhibiting proteic ADP-ribosilation between 1 and 200 mg/dose.
